(19) Europäisches Patentamt
European Patent Office
Office européen des brevets

(11) EP 0 799 623 A2

(12) EUROPEAN PATENT APPLICATION

(43) Date of publication:
08.10.1997 Bulletin 1997/41

(51) Int Cl.6: A61L 29/00

(21) Application number: 97302296.5

(22) Date of filing: 03.04.1997

(84) Designated Contracting States:
BE DE ES FI FR GB NL SE

(30) Priority: 04.04.1996 GB 9607201

(71) Applicant: The BOC Group plc
Windlesham Surrey GU20 6HJ (GB)

(72) Inventor: Larsson, Nils Evert
253 73 Helsingborg (SE)

(74) Representative: Wickham, Michael et al
c/o Patent and Trademark Department
The BOC Group plc
Chertsey Road
Windlesham Surrey GU20 6HJ (GB)

# (54) Medical article with a lubricious coating

(57) A method of making a medical article, for example a central venous catheter, able to release an anti-microbial agent in vivo comprising:

i) forming on at least the external surface of a polyurethane member a hydrophilic coating which becomes lubricious on contact with aqueous liquid;

ii) contacting the coated member with an aqueous solution of benzalkonium halide or other anti-microbial agent so as to impregnate the coating with said halide;

iii) drying the impregnated coating; and

iv) immersing the thus treated polyurethane member in an humid atmosphere for a period of at least 30 minutes whereby redistribution of the benzalkonium halide or other anti-microbial agent takes place within the coating;

EP 0 799 623 A2

## Description

The present invention relates to a process for the preparation of a medical article, particularly a medical article, for example a catheter having a lubricious coating impregnated with benzalkonium halide or other anti-microbial agent.

There is an extensive body of publications concerning various methods whereby shaped medical articles, such as catheters, are rendered anti-microbial by the incorporation therein or thereon of a suitable anti-microbial agent or agents.

For example, US Patent No 4,863,444 discloses mixing of the anti-microbial agent with the monomer prior to polymerisation. The resulting polymer is formed into the desired shape either during polymerisation or thereafter. US Patent No 4,479,795 is typical of those patent publications wherein the anti-microbial agent is mixed with the polymer before curing. Difficulties would however arise in achieving satisfactory polymerisation without degradation of the anti-microbial agent if such methods were to be practised with an anti-microbial agent comprising a benzalkonium halide.

US Patent No 5,013,306 is representative of a substantial number of patent publications wherein the anti-microbial agent is combined with the polymer and extruded to form the desired medical article having the anti-microbial agent distributed throughout. By utilising polymer mixtures containing varying amounts of anti-microbial agent, shaped articles, such as catheters, can be formed by coextrusion techniques to have layers containing differing concentrations of the anti-microbial agent. Such combination of the polymer with a benzalkonium halide is not however possible without the degradation problem arising.

Accordingly, EP-A-0 520 160 discloses a method of forming an anti-microbial catheter by contacting a polyurethane catheter with a solution of benzalkonium halide or similar anti-microbial agent in a chlorinated or fluorinated hydrocarbon, particularly methylene chloride.

It is generally recognised as desirable for a polyurethane catheter to be given a coating of a lubricious material by which is meant a material which is lubricious when exposed to an aqueous medium. Therefore, EP-A-0 520 160 recommends coating with a layer of the lubricious material the devices treated with the benzalkonium halide anti-microbial agent. In other words, the polyurethane catheter is impregnated with the anti-microbial agent and only then coated with the layer of the lubricious material. One reason for adopting such a procedure is that there is a tendency for ionic benzalkonium halide to be released in vivo from the catheter relatively rapidly if the benzalkonium halide is incorporated into the coating at the surface of the catheter, thereby reducing the period of time over which the catheter is able to give effective anti-microbial protection. By impregnating the polyurethane catheter with the anti-microbial agent, however, and then coating, the problem of too rapid a release of anti-microbial agent is avoided.

We have further discovered that such solvents as methylene chloride can compromise the integrity of thin walls that are typically present in plural lumen catheters. Accordingly, in some instances, the method according to EP-A-0 520 160 is unsuitable for commercial use.

We have yet further discovered that the presence of benzalkonium halide between a polyurethane catheter and a lubricious coating may compromise the bond between the catheter and the coating. Accordingly, the mere substitution of a different solvent for the methylene chloride is not a satisfactory solution to the problems we have discovered.

Indeed, it can be appreciated from the foregoing that a major problem exists in providing on a commercial basis a range of catheters all impregnated with an anti-microbial agent comprising benzalkonium chloride. The agent is unsuitable for incorporation into polyurethane during the initial extrusion of a hollow or tubular polyurethane material. Use of a methylene chloride impregnating solution attacks the polyurethane catheter itself. If it is attempted to introduce the anti-microbial agent into the catheter between the polyurethane member and the lubricious coating the benzalkonium halide interferes with the bonding of the catheter to the lubricious coating. If it is attempted to impregnate the catheter after coating, the duration of effective anti-microbial action is reduced.

It is an aim of the present invention to provide a method of making a medical article able to mitigate these difficulties.

According to the present invention there is provided a method of making a medical article able to release an anti-microbial agent in vivo comprising:

i) forming on at least the external surface of a polyurethane member a hydrophilic coating which becomes lubricious on contact with aqueous liquid;

ii) contacting the coated member with an aqueous solution of benzalkonium halide or other anti-microbial agent so as to impregnate the coating with said halide;

iii) drying the impregnated coating; and

iv) immersing the thus treated polyurethane member in an humid atmosphere for a period of at least 30 minutes whereby redistribution of the benzalkonium halide takes place within the coating.

As a result of the redistribution of the benzalkonium halide in the coating, the duration of effective anti-microbial protection provided by the benzalkonium halide is increased. The phenomenon of redistribution is wholly unexpected and provides a substantial improvement in the anti-microbial performance of the catheter in vivo.

Preferably, the said period is of sufficient duration for an uniform distribution of benzalkonium halide in the coating to be achieved. Elevated temperature accelerates the redistribution. Preferably, a temperature in the range of 45 to 70°C is employed. If such a temperature is chosen, the period of immersion is in the range of 3 to 5 hours. Lower temperatures make desirable longer periods of immersion in order to achieve an uniform distribution of the benzalkonium halide in the coating.

The invention also provides a medical article made by the method according to the invention.

The method according to the invention is particularly suitable for use in making single or plural lumen catheters, for example venous catheters.

Typically, a finished catheter made according to the invention contains from 30 to 260 (preferably 100 to 200) microgrammes of benzalkonium halide per centimetre of coated length depending on the gauge of the catheter and the number of lumens it employs.

The actual amount of benzalkonium halide incorporated into the coated polyurethane member can be controlled by appropriate selection of the concentration of benzalkonium halide in the impregnating solution the thickness and viscosity of the hydrophilic layer and the length of the period of time throughout which the polyurethane member is in contact with the impregnating solution. Typically, we have found that a period of time of from 5 to 60 seconds (more typically 15 to 50 seconds) and a concentration of benzalkonium halide in the solution of from l to 10% by weight typically 2 to 4% by weight are suitable.

Preferred values of these parameters depend upon the number of lumens or passages in the catheter and the external diameter (gauge) of the catheter. In selecting the impregnation time and the concentration of the impregnating solution, it needs to be borne in mind that if the weight of benzalkonium halide per unit length of catheter is too low the rate of release of benzalkonium halide in vivo is too low to be efficacious clinically as an anti-microbial agent, but if the weight of benzalkonium halide per unit length of catheter is too high the rate of release of benzalkonium halide, in vivo may be sufficiently great to cause mild haemolysis. Within these limits there is sufficient room to be able readily to select an impregnating time and concentration of impregnating solution so as to enable there to be made an anti-microbial catheter which is able to release anti-microbial agent at an effective rate in vivo without causing any haemolysis.

By so selecting the impregnation time and concentration of the impregnating solution between about 30% and about 45% (typically between 38% and 42%) of the benzalkonium halide content of a catheter can be released over about the first 24 hours in vivo. It is during such an initial twenty four hour period that there is the greatest risk of infection from bacteria that may come to colonise the catheter. The rate of release slows thereafter. Typically, there is a continual release from the catheter of an effective concentration of benzalkonium halide over a period of seven to ten days. In the absence of the sterilisation step from 60 to 70% of the benzalkonium halide would be released within the first 24 hours with the result that the period over which there is effective release of benzalkonium halide would be reduced.

The article or catheter with the dried coating is preferably sterilised in an humid atmosphere. There is therefore typically no need to have discrete immersion and sterilising steps because the sterilising atmosphere and time can be selected so that the necessary redistribution of the benzalkonium chloride takes place during sterilisation, the choice of sterilising atmosphere and sterilising time is not critical to the invention. It is however desirable that each batch of impregnated articles according to the invention be sterilised under the same conditions with the result that the release pattern of benzalkonium halide in vivo is constant from batch to batch. The sterilising gas mixture preferably contains gas mixture comprises a mixture of steam, ethylene oxide and non-reactive gas. The nonreactive gas may be carbon dioxide or a fluorocarbon (e.g. R134 or R123a). The sterilising temperature is typically in the range of 45 to 70°C. The sterilising time is typically in the range of 3 to 5 hours.

Alternatively, discrete immersion and sterilising steps may be performed.

The polyurethane employed to make the polyurethane member is typically prepared by the condensation reaction of an aromatic or aliphatic polyisocyanate (such as toluene diisocyanate) and a plural functional hydroxyl-containing material such a phenol, alcohol, amino alcohol or carboxylic acid (hereinafter referred to collectively as "polyols"). The basic polyurethane polymer unit is formed by the following type of reaction:

$$R_1NCO + R_2OH \text{ ------> } R_1NHCOOR_2$$

Useful polysiocyanates in the above reaction include; toluene-2,3-diisocyanate, toluene-2,6-diisocyanate; commercial mixtures of toluene-2,4 and 2,6-diisocyanates; cyclohexylene-l,4-diisocyanate; m-phenylene diisocyanatodiphenylether; 2,4-dimethyl-1,3-phenylene diisocyanate; 4,4-diisocyanatodiphenylether; and 2,4,5-toluene triisocyanate.

Useful polyols in the above reaction include polyester polyols obtained form the condensation of polycarboxylic acids, preferably dicarboxylic acids, such as adipic, phthalic, oxalic, malonic, cyclohexane-1,2-dicarboylic, naphthalene-l,2-dicarboxylic, fumaric, itaconic acid and the like, with polyols, preferably dials, such as ethylene glycol, diethylene glycol, sorbitol, triethanolamine, di)beta-hydroxyethyl)ether, and the like, and amino alcohols such as ethanolamine, 3-aminopropanol, 10-aminodecanol, 6-amino-5-methylhexanol-1, p-hydroxymethylbenzylamine, and the like. Polyesters derived from ring-opening or condensation of lactones with polyfunctional compounds such as any of the polyalcohols set out above can also be used to provide the thermoplastic polyurethane polymers of the present invention.

Examples of preferred thermoplastic physiologically acceptable polyurethanes for use in accordance with the present invention include: polytetramethylene ether glycol diphenylmethane diisocyanate (MDI); polytetramethylene ether glycol toluene diisocyanate (TDI); poly(1,4-oxybutylene) glycol diphenylmethane diisocyanate; polypropylene glycol toluene diisocyanate; polyethylene adipate diphenylmethane diisocyanate; polyethylenepolypropylene adipate isophorone isocyanate polyurethanes; and the like, and mixtures thereof. Preferred physiologically acceptable polyurethanes include Pellethane® series 2363 available from Dow Chemical Company, Tecothane®, commercially available from Thenodics. Estane®, commercially available from B F Goodwich, Texin®, commercially available from Mobay Chemical Co and the like. In a most preferred embodiment, the physiologically acceptable polyurethane is Pellethane® series 2363, which is a polyether polyurethane synthesized from methylene bis-(p-phenyl isocyanate) polytetramethylene glycols and 1,4-butanediol.

Although polyurethanes are generally linear, in order to provide solubility and thermoplasticity, they can be cross-linked by adding a sufficient quantity of a crosslinking agent to a solvent solution of the polymer or by incorporating the crosslinking agent into a melt polymer mixture while it is still in the plastic state. Examples of such crosslinking agents are isocyanates, polycarboxylic acids, peroxides, organo-titanates, and the like.

Chain extenders with hydrogen-containing difunctional compounds such as water, diamines, or amino acids may also be used. Useful chain extenders include 1,4-butanediol, hexamethylene diamine, 4,4-methylene-bis-(2-chloroaniline) (MOCA), trimethylolpropane, and ethanolamine. Other useful additives include accelerators, catalysts, stabilisers, plasticizers, and the like which improve or modify the properties of the polyurethane. Examples of such useful additives include dicumyl peroxide, benzothiazole disulfide, polypropylene adipate, and metal salts such as potassium acetate, cobalt naphthenate and zinc chloride.

The benzalkonium halide may be selected from any compounds falling within the scope of formula (I) below:

$$[C_6H_5NR_1R_2R_3]^+ X^- \qquad (I)$$

wherein $R_1$ and $R_2$ are methyl groups and $R_3$ is a higher alkyl group and X is a halogen. The benzyl group may contain substituents (e.g. one or more chlorine radicals) or may be unsubstituted. The benzalkonium halide may be selected from any compounds falling within the scope of formula I or from mixtures of such compounds. The benzalkonium halide may typically comprise a mixture of the halide salts of alkyl dimethyl benzyl ammonium compounds, wherein the alkyl group is a mixture of groups comprising from 8 to 18 carbon atoms; or alkyl dimethyl dichlorobenzyl ammonium compounds. Preferred benzalkonium compounds are selected from the group consisting of tetradecyl dimethyl benzyl ammonium halide, hexadecyl (cetyl) dimethyl benzyl ammonium halide, and octadecyl (stearyl) dimethyl benzyl ammonium halide and mixtures thereof. preferred halide is the chloride. Benzalkonium halides have demonstrated a wide range of antibacterial activity against both Gram-positive and Gram-negative bacteria and yeasts. They have demonstrated good activity against those organisms commarily associated with central venous catheter sepsis, particularly *Staphylcoccus epidermis,* the most prevalent cause, and others including *Staphylcoccus aureus, Escherichia cali,* and certain *Klebsiella, Proteus* and *Candidia* species.

The polyurethane members for use in the present invention may be made by any means conventional in the art, e.g. extrusion, casting, and injection moulding, with injection moulding being preferred. The catheters may be of any standard gauge and be either single or plural lumen.

The polyurethane member is coated with a layer which becomes lubricious when it comes in contact with body fluids, the coating preferably being applied by immersion in a coating solution which comprises a hydrophilic poly(N-vinyllactam) in a solvent comprising a mixture of methyl ethyl ketone, ethyl lactate and trichloroethylene. The term "poly(N-vinyl lactam)" as used herein means physiologically acceptable homopolymers and copolymers of such N-vinyl lactams as N-vinylpyrrolidone, N-vinylbutyrolactam, N-vinylcaprolactam, and the like, and mixtures thereof.

In a preferred embodiment, the poly(N-vinyllactam) is polyvinylpyrrolidone (PUP) homopolymer preferably having a weight average molecular weight of about 1,100,000, the coating layer solution comprises from about 1% to about 10%, preferably about 2.5% to 5%, by weight of PVP. In general, the coating layer solution is prepared by dissolving the poly (N-vinyl lactam) in the solvent at room temperature. The solution is contacted with the catheter by conventional means such as dipping, spraying, or the like. The excess coating solution is then removed and the coated polyurethane members are dried in an oven to remove the solvent at an elevated temperature.

The lubricious coating is preferably impregnated with benzalkonium halide in accordance with the present invention by immersion in an aqueous solution thereof at ambient temperature.

A preferred immersion procedure in accordance with the present invention is a 30 second immersion in a 3% by weight aqueous solution of benzalkonium halide, preferably benzalkonium chloride. After immersion,

the catheters are immediately removed from the solution, drained, heated and maintained at an elevated temperature, preferably a temperature of from about 50° to 60°C for from 20 to 60 minutes.

The catheters impregnated with benzalkonium halide in accordance with the present invention are unexpectedly advantageous in that, as previously stated, they release from about 30% to about 42% of the benzalkonium halide within about 24 hours of initial contact with body fluids and then gradually release the remainder over the ensuing 7 to 10 days. The subject catheters have demonstrated effective anti-microbial activity over a period as long as 14 days from initial contact with body fluids. This release pattern could not have been predicted from the literature, particularly US Patent No 4,769,014, since it would have been assumed therefrom that benzalkonium halide would have been complexed with the PVP in the lubricious coating and released as a complex. It has been determined that benzalkonium halide is not released from catheters treated in accordance with the subject invention as a complex.

The anti-microbial properties of benzalkonium halide-impregnated catheters are reported by Tebbs et al J Antimicrob Chemother (England), Feb 1993, 31,(3), pp 261-271, and Eur J Clin Microbiol Infect Dis, Feb 1994, 13(2), pp 111-117.

The catheters described therein were prepared by a method according to EP-A-520 160.

The method according to the present invention will now be described with reference to the following example:

Example

A triple lumen, 14 gauge, tubular member, 20cm in length, of Dow Pellethane 2363 polyurethane was given a polyvinylpyrrolidone (PUP) coating over essentially its entire length by immersion in a solution of the PVP containing 2.8% by weight of solids. (The solvent comprises a mixture of methyl ethylketone, trichloroethylene and ethyl lactate.) The polyurethane member was held immersed in the coating solution for sixty seconds at 25°C and a pressure of 1 bar. The resulting coated member was dried in an oven for 1 hour at a temperature of 55°C.

The resulting coated catheter was then immersed in a diluted aqueous solution of STEPAN BTC 50 USP (trade mark) (available from Stepan Company, Northfield, Illinois 60093, United States of America) antimicrobial agent containing 3% by weight of active ingredients. The active ingredients were all of formula:

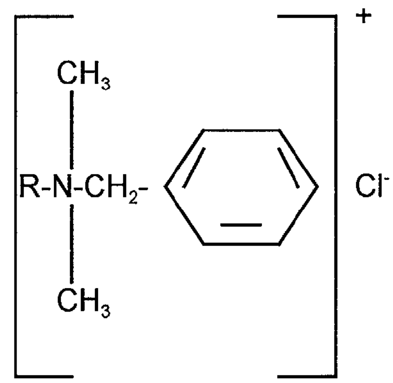

where R was a higher alkyl group. In 50% by weight of the active ingredients R was a $C_{18}H_{25}$, group; in 30% a $C_{14}H_{29}$ group; in 17% a $C_{16}H_{33}$ group, and in 3% a $C_{18}H_{37}$ group. The catheter was held immersed for 30 seconds at 25°C and 1 bar. The resulting benzalkonium chloride impregnated catheter was dried in an hour for 1 hour at a temperature of 55°C and a pressure of 1 bar. The resulting catheter was found to have taken up 194 microgrammes of benzalkonium chloride/em of the catheter.

The impregnated catheter was then sterilised in a chamber for four hours in a humid atmosphere of a sterilising gas mixture consisting of 75% by volume of ethylene oxide and 25% by volume of carbon dioxide at a temperature of 50% by volume of carbon dioxide at a temperature of 50°C and a pressure of 85 kPa. (The humidity was present in the atmosphere for only three and a quarter hours of the four hour sterilising period.) It was found that after sterilisation the amount of benzalkonium chloride released by the catheter in the first 24 hours was approximately 50% of that predicted from experiments performed with unsterilised catheters.

The clinical performance of catheters prepared in accordance with this example has been such as to demonstrate a zone of inhibition of Staphylococcus Epidermidis equal to 15 mm after insertion in patients for three days.

The assay is proposed by inoculating nutrient agar with organisms and placing the catheter section into the inoculated agar, incubating for 24 hours and then measuring the zone of inhibition around the catheter.

The catheter was inserted in the patient for 3 days, removed and then assayed.

**Claims**

1. A method of making a medical article able to release an anti-microbial agent in vivo comprising:

   i) forming on at least the external surface of a polyurethane member a hydrophilic coating which becomes lubricious on contact with

aqueous liquid;

ii) contacting the coated member with an aqueous solution of benzalkonium halide or other anti-microbial agent so as to impregnate the coating with said halide;

iii) drying the impregnated coating; and

iv) immersing the thus treated polyurethane member in an humid atmosphere for a period of at least 30 minutes whereby redistribution of the benzalkonium halide or other anti-microbial agent takes place within the coating;

**2.** A method as claimed in claim 1, wherein the period of immersion is sufficient for an uniform distribution of the benzalkonium halide in the coating to be achieved.

**3.** A method as claimed in claim 1 or claim 2, wherein the article is a catheter.

**4.** A method as claimed in any one of the preceding claims, in which the finished catheter contains from 30 to 260 microgrammes of benzalkonium halide per centimetre of coated length.

**5.** A method as claimed in any one of the preceding claims, in which the coated polyurethane member is impregnated by immersion in an aqueous impregnating solution of said benzalkonium halide.

**6.** A method as claimed in claim 5, wherein a chosen length of the polyurethane member is held immersed in the impregnating solution for from 5 to 60 seconds.

**7.** A method as claimed in claim 5 or claim 6, wherein said impregnating solution contains from 2 to 4% by weight of benzalkonium halide.

**8.** A method as claimed in any one of the preceding claims, wherein the atmosphere has a temperature in the range of 45 to 70°C.

**9.** A method as claimed in any one of the preceding claims, wherein the humid atmosphere is a atmosphere comprising ethylene oxide.

**10.** A method as claimed in any one of the preceding claims, wherein the benzalkonium halide anti-microbial agent is selected from compounds having the formula:

$$[C_6H_5NR_1R_2R_3]^+ X^-$$

wherein $R_1$ and $R_2$ are both a methyl group, $R_3$ is a higher alkyl group having from 8 to 18 carbon atoms, and X is a halogen, and mixtures thereof.

**11.** A method as claimed in claim 10, wherein the benzalkonium halide ntimicrobial agent is selected from tetradecyl dimethyl benzyl ammonium halide; hexadecyl dimethyl benzyl ammonium halide; and octadecyl dimethyl ammonium halide.

**12.** A method as claimed in claim 10 or claim 11, wherein the or each halide is a chloride.

**13.** A method as claimed in any one of the preceding claims, wherein the lubricious coating comprises a polyvinylpyrollidone homopolymer.